# EUROPEAN PATENT APPLICATION

(11) **EP 2 500 054 A1**
(43) Date of publication of application: **19.09.2012**
(21) Application number: 11800895.2
(22) Date of filing: 29.06.2011
(51) Int. Cl.: A61M 15/02, A61M 11/02, A61M 16/06

(54) **GAS MIST INHALER**

(30) Priority: 01.07.2010 JP 2010150848
(71) Applicant: Nakamura, Shoichi, Higashichikuma-gun, Nagano 399-7502 (JP); ACP Japan Co. Ltd., Tokyo 113-0033 (JP)
(72) Inventor: SHOICHI, Nakamura, Higashichikuma-gun, Nagano 399-7502 (JP)
(74) Representative: Gill, David Alan
(86) International application number: PCT/JP2011/064910
(87) International publication number: WO 2012/002433

(57) **Abstract**

The present invention is to offer a gas mist inhaler which is simple and has excellent effects by using physiological actions of carbon dioxide or oxygen. The gas mist inhaler comprises a gas supply means 110 of supplying oxygen, carbon dioxide, or mixed gas of oxygen and carbon dioxide, a gas mist generation means 120 connected to the gas supply means 110 as well as having a liquid storage of storing a liquid, a nozzle of supplying gas under pressure, a liquid suction pipe of sending the liquid to the front end of the nozzle, and an air hole 127 of taking in outside air; the gas mist inhaler further comprises a gas mist generation means 120 of generating a mist prepared by pulverizing and dissolving the liquid and gas; and an inhalation member 140 connected to the gas mist generation means 120 and having an inhalation port of inhaling the gas mist into the living organism, the above mentioned gas mist generation means 120 further has a gas introduction means of supplying gas into the gas mist generation means 120, independently of the above mentioned nozzle, thereby to heighten the supplying pressure of the gas mist into the inhalation member 140.

## Description

### Technical Field

The present invention relates to a gas mist inhaler for carrying out oral inhalation of a gas mist into a living organism, and the gas mist is prepared by pulverizing and dissolving oxygen, carbon dioxide or mixed gas of oxygen and carbon dioxide, and liquid of medicines.

### Background Art

Conventionally, it has been known that carbon dioxide (carbonic acid anhydride: CO₂) has two properties of being not only soluble in water (water-soluble) but also soluble in fat (fat-soluble) and, owing to having both properties, when only contacting to the skin and mucous membrane of the living organism being as mixed with water and fat, carbon dioxide well penetrates under a subcutaneous layer and expands blood vessels around the parts of penetrated carbon dioxide, and it works to improve the blood circulation. By the action of these accelerating the blood circulation, it displays various physiological effects such as dropping of blood pressure, improving of metabolism or accelerating to remove pain substance or waste product. Further, it has also anti- inflammation and anti-bacterial. Therefore, carbon dioxide has recently been given attentions also from viewpoints of improving health or beauty other than the purpose of medical cares.

In the organization of the living organism, carbon dioxide works to release oxygen having been carried in combination with hemoglobin in a red blood cell. Around parts at the high concentration of carbon dioxide, the red blood cell releases more oxygen. Thus, supply of oxygen to cells by the red blood cell is mainly controlled by carbon dioxide. In short, being without carbon dioxide, hemoglobin remains as having been combined with oxygen and the cell becomes unable to receive oxygen. Carbon dioxide seems to be waste products resulted from action of the cell, however, as is seen, it plays in fact very important roles in the living organism.

Further, recently, oxygen of the high concentration has also widely been known as effective over activity of metabolism, acceleration of blood circulation, fatigue recovery, or stability of blood pressure. Other than them, oxygen has effects of disinfection or sterilization by oxidation.

By the way, for easing a symptom of disease in a respiratory system such as asthma or allergic rhinitis, oral nasogastric inhalation of medicines or steam using an inhaler or a nose spray have been till now carried out.

A nasogastric vaccine (spray system collunarium vaccine) has recently developed for forming a mucous immunity against an influenza virus, and its high effect has been given attention. The nasogastric vaccine is generally higher in effects of preventing crises of influenza than the vaccine injected under the skin, and has a merit of effectively working against a influenza stock. In addition, also in prevention or cure to the influenza, an antiviral drug of inhalation dosage has been developed.

### Summary of Invention

### Problems to be solved by the Invention

However, there has never been such an inhaler which causes carbon dioxide or oxygen to be efficiently dissolved in the above mentioned medicines, and can effectively influence a physiological action to a living organism in addition to the medicine.

Accordingly, in view of these circumstances, it is an object of the invention to provide a gas mist inhaler being simple and excellent in effects by using physiological actions of carbon dioxide or oxygen.

### Means for solving the Problems

For solving the above mentioned problems, the invention is to provide a gas mist inhaler which comprises a gas supply means of supplying oxygen, carbon dioxide, or mixed gas (called as "gas" hereafter) of oxygen and carbon dioxide; a gas mist generation means connected to the gas supply means as well as having a liquid storage of storing a liquid, a nozzle of supplying gas under pressure, a liquid suction pipe of sending the liquid to the front end of the nozzle, and an air hole of taking in outside air, the above mentioned gas mist generation means of generating a mist (called as "gas mist" hereafter) prepared by pulverizing and dissolving the liquid and gas; and furnishing an inhalation member connected to the gas mist generation means and having an inhalation port of inhaling the gas mist into the living organism, the above mentioned gas mist generation means further having a gas introduction means of supplying gas into the gas mist generation means, independently of the above mentioned nozzle, characterized by heightening supplying pressure of the gas mist into the inhalation member.

By the way, the invention refers it as "pulverizing and dissolving" to pulverize the liquid into fine liquid drops, and cause to contact and mix with gas (oxygen or carbon dioxide, or mixed gas of oxygen and carbon dioxide).

Herein, preferably, the gas mist generation means further has an air hole for taking in outside air, otherwise it is also sufficient for the inhalation member to have a further opening for taking in outside air.

The gas supply means is desirably a gas bomb of a cartridge system. Further, the gas supply means may have any one or plurality of a gas supply time setting portion, a gas supply pressure adjusting portion and a gas mixing ratio setting portion.

In addition, the gas mist generation means also may supply the gas mist into the plural inhalation members.

Next, it is desirable that the above mentioned liquid is any one or plural combination of water, ionic water, ozone water, physiological salt solution, purified water, or sterilized and purified water. Desirably, the above medicines are any one or plural combination of menthol, vitamin E, vitamin C derivative, retinol, anesthetic agent, cyclodextrin, photo catalyst, complex of photo catalyst and apatite, hyaluronic acid, coenzyme Q10, seed oil, propolith, ethanol, chlorhexidine gluconate, amphoteric surface active agent, benzalkonium chloride, alkyl diamino etherglycine acetate, sodium hypochlorite, peracetic acid, sodium sesquicarbonate, silica, povidone-iodine, sodium hydrogen carbonate, carbonate spring agent of high density, anti-allergic agent, anti-inflammatory agent, anti-febrile agent, anti-fungus agent, anti-influenza virus agent, influenza vaccine, steroid agent, anti-cancer agent, anti-hypertensive agent, cosmetic, and tricogen.

A size of the mist supplied from the gas mist generation means into the inhalation member is suitably not larger than 10 µm.

Desirably, the gas mist generating means has a gas mist supply pipe for supplying the gas mist into the inhalation member, and this gas mist supply pipe is preferably furnished with a filter for removing liquid drops attached to the inside of the pipe. Still further, the gas mist supply pipe is suitably composed of a cornice shaped pipe over a whole or at one part of the gas mist supply pipe. In addition, this gas mist supply pipe is provided with a check valve.

Preferably, the gas mist generating means is in advance sterilized.

### Advantageous Effects of Invention

According to the gas mist inhaler of the invention, adding to ordinary effects of the inhaler, by the physiological action of the gas mist, not only permeating a liquid medicine into the upper and lower airways of the living organism, but also activating a blood flow around a diseased part, the invention can display synergistic effects of carbon dioxide or oxygen such as flourishing the blood flow of the disease, rapidly moderating an inflammation or increasing immunological force.

### Brief Description of Drawings

[FIG. 1] A generally schematic view of the gas mist inhaler depending on the first embodiment of the invention;
[FIG. 2] A typical cross sectional view showing the structure of the gas mist generator of the gas mist inhaler of the invention; [FIG. 3] A perspective view, partially in section, of the generator main body of the gas mist generator shown in FIG. 2;
[FIG. 4] A typical cross sectional view of generator main body in the gas mist generator shown in FIG. 2;
[FIG. 5] A perspective view of the middle member in the gas mist generator shown in FIG. 2;
[FIG. 6] A top view and a cross sectional view of the middle member in the gas mist generator shown in FIG. 2;
[FIG. 7] Typical views showing other examples of the inhalation members according to the gas mist inhaler of the invention; [FIG. 8] A typical view showing the example of the gas mist supply pipe connecting the gas mist generator to the inhalation member of the invention;
[FIG. 9] A generally schematic view of the gas mist inhaler depending on a second embodiment of the invention; and
[FIG. 10] A generally schematic view of the gas mist inhaler depending on a third embodiment of the invention.

### Description of Embodiments

In the following description, explanations will be made to embodiments of this invention, referring to the attached drawings.

### [First Embodiment]

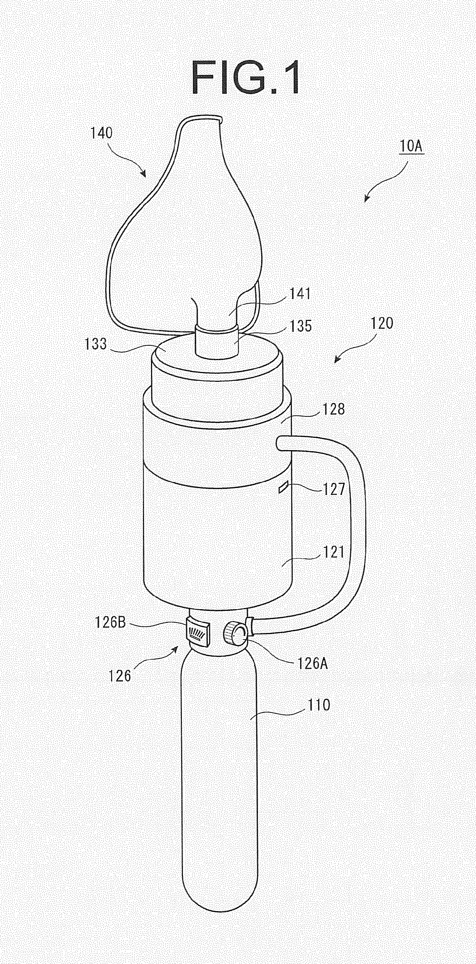

FIG. 1 is the generally schematic view of the gas mist inhaler depending on the first embodiment of the invention.

As showing in FIG. 1, the gas mist inhaler 10A of the present embodiment has a gas bomb 110 as a gas supply means of supplying oxygen, carbon dioxide or mixed gas (called briefly as "gas" hereafter) of oxygen and carbon dioxide, a gas mist generator 120 as a gas mist generating means and an inhalation mask 140 as an inhalation member.

The gas bomb 110 of supplying gas into gas mist generator 120 employs a small sized cartridge system by paying attention to portability in the present embodiment. This small sized gas bomb 110 is, as shown in FIG. 1, attached to a gas bomb connecting portion 126 of the gas mist generator 120 for supplying pre-selected gas into the gas mist generator 120 at predetermined pressure.

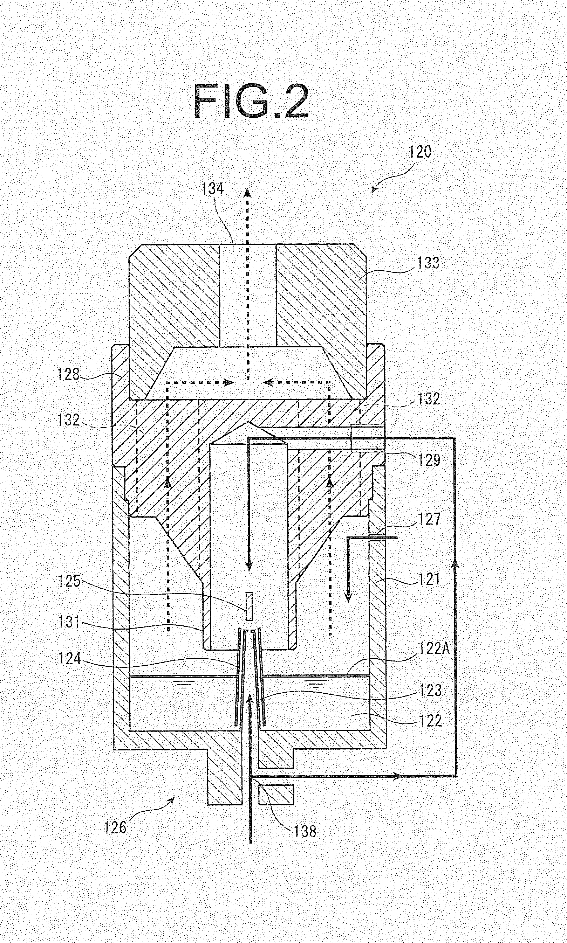

The gas mist generator 120 stores inside a liquid, generates a gas mist prepared by pulverizing and dissolving this liquid and gas by highly flowing gas from the gas bomb 110, and supplies the gas mist into the inhalation mask 140. FIG. 2 is the typical cross sectional view showing the structure of the gas mist generator 120. As shown in FIG. 2, the gas mist generator 120 is composed with a generator main body 121, a middle member 128 and a cover member 133. By the way, preferably, this gas mist generator 120 is in advance performed with a sterilization treatment.

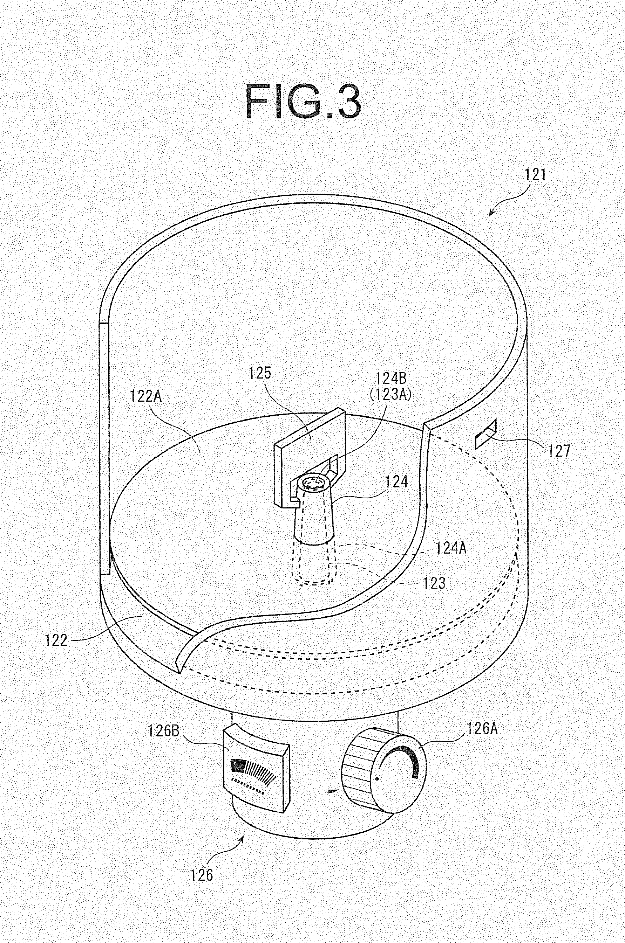

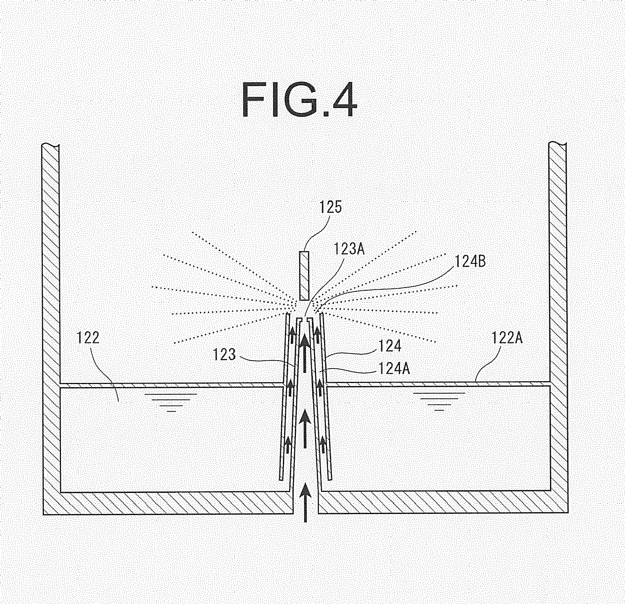

The details of the generator main body 121 are shown in FIG. s 3 and 4. FIG. 3 is the perspective view, partially in section, of the generator main body 121, and FIG. 4 is the cross sectional and typical view of the same. As shown therein, the generator main body 121 is furnished with a liquid storage 122 storing the liquid therein, a nozzle 123 discharging gas supplied from the gas bomb 110 from a nozzle front end opening 123A, a liquid suction pipe forming member 124 forming a liquid suction pipe 124A sucking up the liquid stored in the liquid storage 122 up to the front end of the nozzle 123, and a baffle 125 positioned in opposition to the front end opening 123A of the nozzle 123. Further on, the generator main body 121 is formed with an air hole 127 from which outside air is sucked into the generator main body 121.

The liquid storage 122 is formed, as shown in FIG.s 3 and 4, by partitioning the bottom of, one part of the side wall of the generator main body 121 and a shielding sheet 122A. This shielding sheet 122A serves to force the liquid upward of the liquid sucking pipe 124A by maintaining pressure in the liquid storage 122 highly than pressure in the upper part from the shielding sheet 122A. Therefore, the shielding sheet 122A may be positioned fixedly at a predetermined position of an inner wall of the generator main body 121, otherwise may also be vertically movable in response to the level of a liquid surface in the liquid storage 122. Further, depending on magnification of gas pressure discharged from the front end opening 123A of the nozzle 123, the shielding sheet 122A may not be furnished.

At the bottom center of the generator main body 121 (the liquid storage 122), the nozzle 123 is placed. This nozzle 123 passes through the bottom of the liquid storage 122 and a gas bomb connecting portion 126, and is shaped to be almost circular cone squeezed toward an upper side of the generator main body 121. The nozzle 123 is connected, at its base end, to the gas bomb 110, and can discharge gas from its front end opening 123A.

The liquid suction pipe 124A is formed between the outer circumference of the nozzle 123 and the inner circumference of the liquid suction pipe forming member 124 of the almost circular cone being larger by one turn than the nozzle 123. That is, as shown in FIG. 4, by positioning as covering the liquid suction pipe forming member 124 over the nozzle 123, the liquid suction pipe 124A is defined between the outer circumference of the nozzle 123 and the inner circumference of the liquid suction pipe forming member 124. At this time, since a nail shaped projection (not showing) is provided at a base end of the liquid suction pipe forming member 124, a space is formed at a base of the liquid suction pipe forming member 124 and the bottom of the liquid storage 122, so that the liquid stored in the liquid storage 122 is sucked up from this space by the liquid suction pipe 124A. In addition, the front end 124B of the liquid suction pipe forming member 124 opens nearly the front end opening 123A of the nozzle 123, and the liquid sucked up by the liquid suction pipe 124A collides against the gas flow discharged from the nozzle 123.

The baffle 125 is a member disposed at a position in opposition to a front end opening 123A of the nozzle 123 and a front end portion 124B of the liquid suction pipe forming member 124, and in the present embodiment, this member 125 is fixedly supported to the liquid suction pipe forming member 124. Otherwise, the baffle 125 is sufficient to be fixedly supported to the shielding sheet 122A and the inside of the generator main body 121. The liquid suction pipe forming member 124 is connected to the shielding sheet 122A at the nearly central part in the vertical directions. The shielding sheet 122A is also connected at its outer circumference to the inside of the generator main body 121. Thus, desirably, the generator main body 121 is formed integrally as a whole.

The gas bomb connecting portion 126 can preferably attach and use a gas bomb 110 by one-touch. Although omitting illustration, the gas bomb connecting portion 126 has inside a regulator for enabling to adjust a gas flowing amount supplied from the gas bomb 110. The gas flow from the gas bomb 110 is diverged into two branches (diverging part 138 in FIG. 2) within the gas bomb connecting portion 126, one being supplied to the nozzle 123 while the other being supplied to a later mentioned gas supply portion 129. By the way, herein shown is an example where the gas bomb connecting portion 126 is furnished with a dial switch 126A and a residual gage 126B. ON/OFF of the gas supply and a supplying amount control are available by turning the dial switch 126A. The residual gage 126B indicates a gas remaining amount of the gas bomb 110.

In the liquid storage 122, preferably, a determined liquid is stored in a previous manufacturing stage (in a case of a disposable type). That is, when actually using the present gas mist inhaler 10, a desirable condition is the liquid having been already stored in the gas mist generator 120. By making disposable, the gas mist can be inhaled hygienically and simply. Or, it is also sufficient to furnish a liquid inlet for pouring and supplementing the liquid into liquid storage 122.

Herein, for the liquid stored in the liquid storage 122, it is preferable to employ water, ionic water, physiological salt solution, ozone water, purified water or sterilized and purified water. Further, these liquids are sufficient to contain medicines useful to users' diseases or symptom. As the medicines, for example, listed are anti-allergic agent, anti-inflammatory, anti-febrile agent, anti-fungus agent, anti-influenza virus agent, anti-influenza vaccine, steroid agent, anti-cancer agent, anti-hypertensive agent, cosmetic agent, or trichogen. Further, these liquids are further possible to generate synergistic effects by coupling with a gas physiological action with single or plurality of menthol having a cooling action; vitamin E accelerating circulation of the blood; vitamin C derivative easily to be absorbed to a skin tissue and having a skin beautifying effect; retinol normalizing a skin heratinizing action and protecting the mucous membrane; anesthetic moderating irritation to the mucous membrane; cyclodextrin removing odor; photocatalysis or a complex of photocatalysis and apatite having disinfection and anti-phlogistic; hyaluronic acid having excellent water holding capacity and a skin moisture retention effect; coenzyme Q10 activating cells and heightening immunization; a seed oil containing anti-oxidation and much nutrient; or propolith having anti-oxidation, anti-fungus, ant-inflummatory agent, pain-killing, anesthetic, and immunity. Otherwise the liquids may be added with ethanol, gluconic acid chlorohexizine, amphoteric surface active agent, benzalkonium chloride, alkyldiamino ether glycin acetate, sodium hypochlorite, acetyl hydroperoxide, sodium sesqui-carbonate, silica, povidone-iodine, sodium hydrogen carbonate. In addition, high density carbonate spring may be added (as examples organic components, sulfate, carbonate, sodium dichloroisocyanurate).

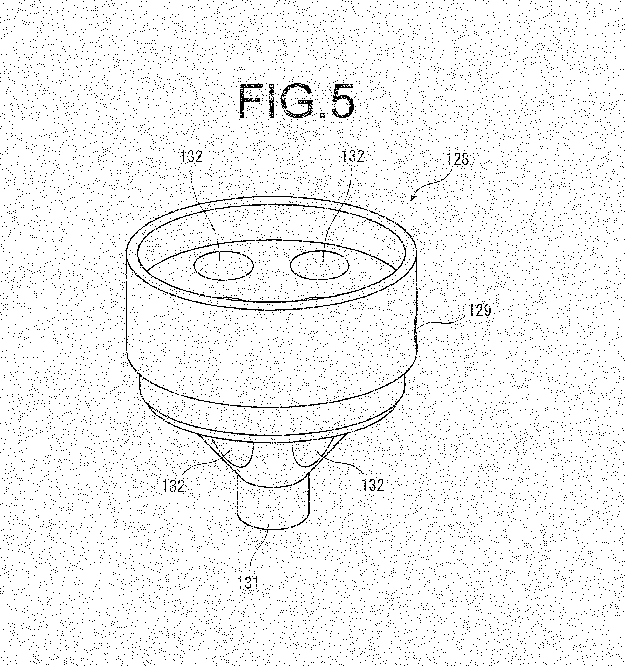

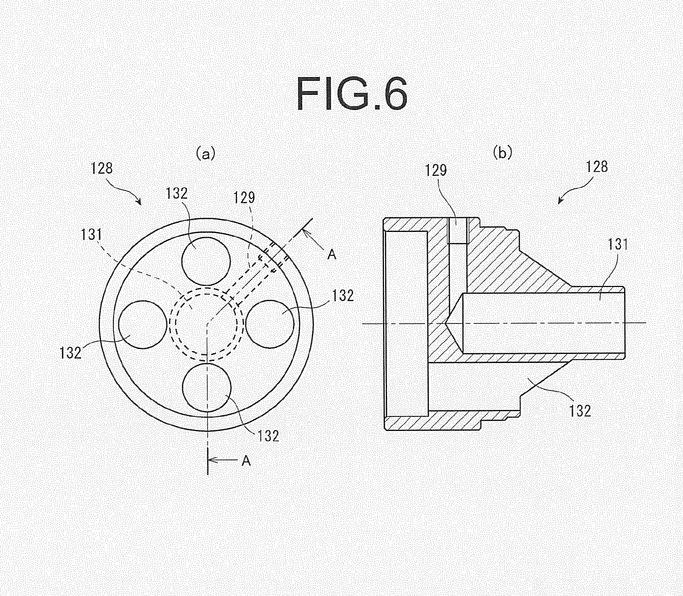

A middle member 128 is positioned at the middle part of the generator main body 121 and a cover member 133, collects the gas mist generated in the generator main body 121, discharges into an inhalation mask 140 and, at the same time, supplies gas into the generator main body 121, thereby to heighten a supplying pressure of the gas mist into the inhalation mask 140. The middle member 128 is shown in detail in FIG. s 5 and 6. FIG. 5 is the perspective view of the middle member 128, and FIG. 6 (a) is a top view of the same, while FIG. 6(b) is the A-A cross sectional view of FIG. 6 (a).

As shown in these illustrations, the middle member 128 is shaped as a spinning top which has a gas supply portion 129 of supplying gas into the gas mist generator 120, a gas inlet 131 of introducing gas into the generator main body 121 as well as producing an air flow discharging the gas mist, and a gas mist collector 132 of collecting the gas mist and discharging it to the inhalation mask 140.

The gas supply portion 129 is a pipe-shaped hole almost horizontally provided and perpendicularly connected with respect to a gas inlet 131. Gas diverged at a gas bomb-connecting portion 126 is supplied into the generator main body 121 via the middle member 128 from the gas supply portion 129. The gas inlet 131 is, as shown in FIG. 2, a pipe-shaped hole which is disposed in the vertical directions extending as covering the vicinity of the gas mist generating parts (a nozzle front end opening 123A, the liquid suction pipe front end portion 124B, and baffle 125), and which introduces gas to the gas mist generator, thereby to exhaust efficiently the gas mist from the inside of the generator main body 121. As is seen, independently of the nozzle 123, a gas introducing means of introducing gas into the gas mist generator 120 is composed of the gas supply portion 129 and the gas inlet 131.

On the other hand, the gas mist collectors 132 are one or a plurality (herein, four, as an example) of pipe shaped holes formed in the vertical positions not crossing with the gas supply portion 129 and the gas inlet 131, and those lead the air current of the gas mist into a gas mist leading part 134 of the cover member 133 positioned on the upper part of the middle member 128, this gas mist being generated by introducing gas from the gas inlet 131 into the generating part of the gas mist.

On the upper part of the middle member 128, the cover member 133 is placed. The cover member 133 is provided with the gas mist leading part 134, to which a gas mist outlet 135 is connected. The gas mist generated in the gas mist generator 120 is supplied into an inhalation mask 140 via a gas mist outlet 135 from the gas mist leading part 134.

The inhalation mask 140 is such an inhalation member having a shape covering a user's respiratory organs (herein, the nose and mouth) for the user to easily breath the generated gas mist. The inhalation mask 140 is connected to the gas mist outlet 135 via the connecting portion 141, and the user breathes the gas mist from the inhalation port 142. Although omitting the illustration, the inhalation mask 140 is preferably formed with an aperture for taking in outside air and avoiding harmful influences by inhalation for a long time of oxygen of high concentration and carbon dioxide.

Next, explanation will be made to a sequence of inhaling the gas mist with the gas mist inhaler 10A of the above mentioned first embodiment.

At first, under a condition of having already stored the liquid in the liquid storage 122 (if not yet stored, such a condition is made of having finished to store), the gas bomb 110 is set to the gas bomb connecting portion 126 of the gas mist generator 120. When turning ON a dial switch 126A of the gas bomb connecting portion 126, gas starts to go into the nozzle 123 and the gas supply portion 129. Incidentally, the dial switch 126A can adjust a gas flowing amount.

When gas is supplied into the nozzle 123, since the nozzle 123 is reduced toward the front end as shown in FIG. 4, gas increases the flowing speed and is exhausted. The liquid is sucked up within the liquid suction pipe 124A owing to negative pressure caused by air flow at this time, is blown up by gas at the front end portion 124B of the liquid suction pump 124A, and collides against the baffle 125, so that the mist is generated. Desirably, the diameter of the mist generated by this collision is fine, and concretely, best is not larger than 10 µm. The thus finely pulverized mist can display effects of minus ion.

Gas is further supplied into the generator main body 121 from the gas supply portion 129 and also from the gas inlet 131, and heightens the exhausting pressure of the generated gas mist. The gas mist is exhausted into the inhalation mask 140 from the gas mist outlet 135. The gas mist exhausted in the inhalation mask 140 can be inhaled from an inhalation port 142.

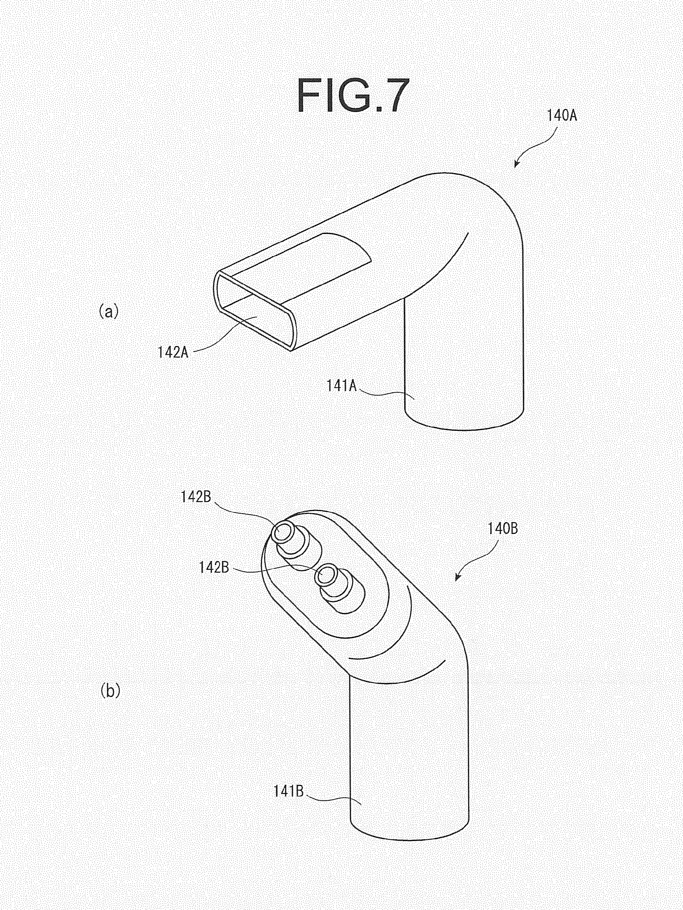

The above reference has showed, as the inhalation member, the example of using the inhalation mask 140 of a type covering the nose and the mouth, and other various types using the inhalation members are available. FIG. 7 shows other examples of the inhalation members.

FIG. 7(a) is a mouth mask 140A of a mouth piece type used for inhaling from the mouth only. At the connecting portion 141A, this is connected to the gas mist outlet 135, and the user inhales the gas mist into the mouth from the inhalation port 142A. The mouth mask 140A is especially suitable to moderate and cure symptoms of the throat.

FIG. 7 (b) is a nose mask 140B of a nose piece type used for inhaling from the nose only. At the connecting portion 141B, this is connected to the gas mist outlet 135, and the user inhales the gas mist into the nose from the inhalation port 142B. The nose mask 140B is especially suitable to moderate and cure allergic rhinitis of the nose.

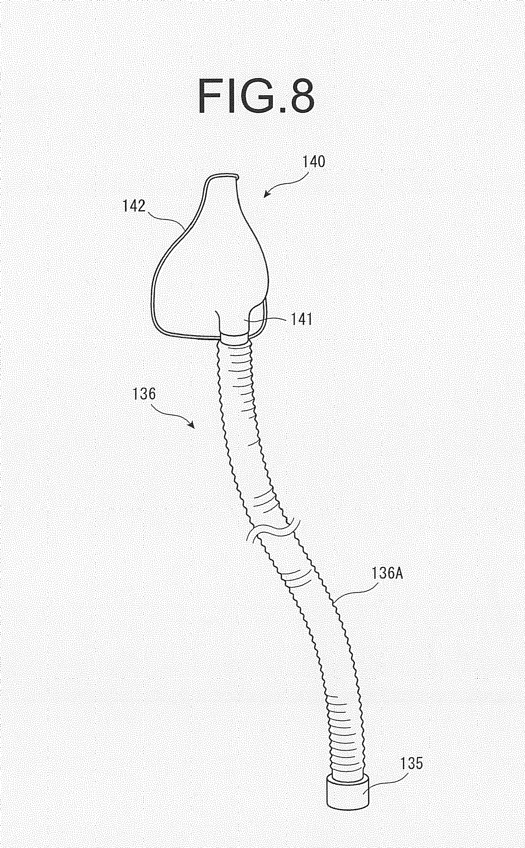

The above reference has shown the example where the gas mist outlet 135 is directly connected to the connecting portion 141 of the inhalation mask 140, and as shown in FIG. 8, the gas mist outlet 135 may be also connected to the inhalation mask 140 via the gas mist supply pipe 136. For such a case, the inside of the gas mist supply pipe 136 is furnished with a check valve for preventing back flow of the gas mist, though not illustrated. The gas mist supply pipe 136 may be provided with a filter for removing surplus liquid drops attached to the inside of the pipe, though not illustrated.

Further, if the gas mist supply pipe 136 is composed wholly or partially with a soft and cornice shaped pipe 136A of large diameter as shown in FIG. 8, it is freely bent or expanded and contracted so that the user's action is not limited. In addition, even if the gas mist flowing in the gas mist supply pipe 136 becomes liquefied, the cornice can remove the liquid owing to its concave and convex parts.

### [Second Embodiment]

The above first embodiment has shown the example as the gas supply means using the small sized gas bomb 110 of the cartridge system, and the present embodiment will show an example using a gas supply device of a stationary type.

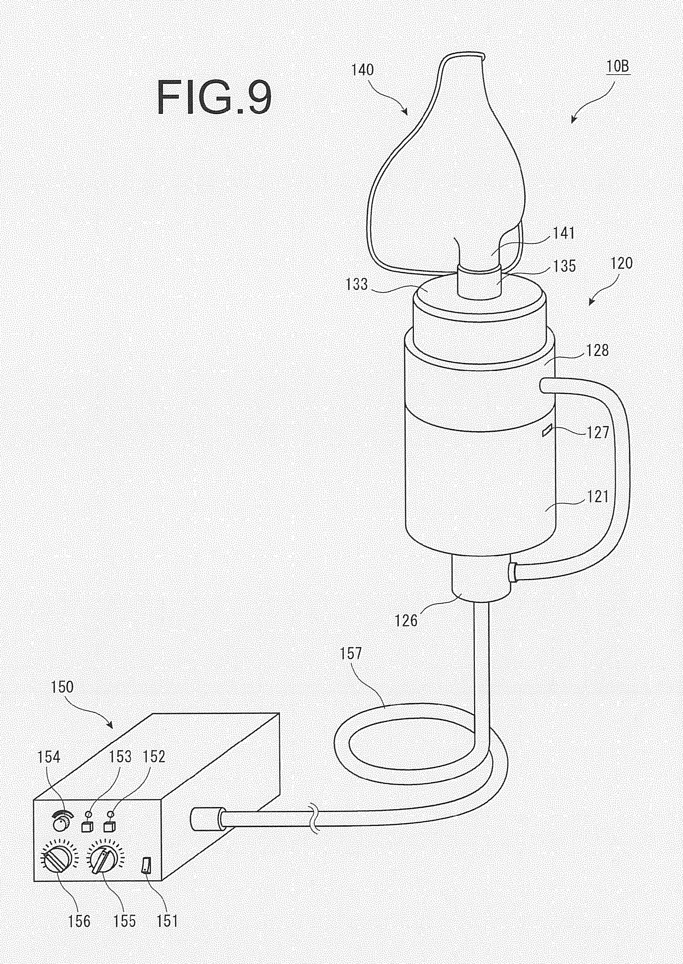

FIG. 9 is the generally schematic view of the gas mist inhaler depending on the second embodiment of this invention. As to the same parts as those of the embodiment shown in FIG.s 1 to 8, the same numerals will be given, and detailed explanation will be omitted.

As shown in FIG. 9, the gas mist inhaler 10B of this embodiment has a gas supply device (gas supply means) 150, the gas mist generator (gas mist generating means) 120 and inhalation mask (inhalation member) 140.

The gas supply device 150 is the stationary typed gas supply device of supplying gas (oxygen, carbon dioxide or mixed gas of oxygen and carbon dioxide) into the gas mist generator 120 at predetermined pressure. Its inside is built-in with the gas bomb (not shown), and may be built-in with a compressor, otherwise may be connected to an external gas bomb.

The gas supply device 150 is, for example as shown in FIG. 9, provided with a power supply switch 151, oxygen supply ON/OFF switch 152, carbon supply ON/OFF switch 153, gas mixing ratio setting part 154, OFF timer (gas supply time setting part) 155, and gas supply pressure adjusting part 156. That is, in the present embodiment, the gas bomb connecting portion has neither the dial switch nor the residual gage (the gas bomb connecting portion 126A), but the gas supply device 150 carries out various controls. The gas supply device 150 and the gas mist generator 120 are connected by the gas supply pipe 157.

The gas mixing ratio setting part 154 is so composed as to regulate voluntarily the mixing ratio of carbon dioxide and oxygen. It is thereby possible to reply at will to the user's requests, for example, for lightening asthma or recovering fatigue such as setting the mixing ratio of oxygen to be much, or for accelerating a blood circulation such as setting the mixing ratio of carbon dioxide to be much. Further, the OFF timer 155 is for setting the gas supplying time, and when a set time passes away, the gas supplying is automatically stopped. The gas supply pressure adjusting part 156 can set the gas supplying pressure arbitrarily. Since the gas supplying time or pressure are possible thereby, its using scope can be broadened.

Incidentally, similarly to the first embodiment, herein illustrated is an example where gas supplied from the gas supply device 150 is diverged into two at the gas bomb connecting portion 126, and instead of this example, such a structure is enough in which gas is directly supplied into the nozzle 123 and the gas supply portion 129 from the gas supply device 150. In such a case, the same gas may be supplied in the nozzle 123 and the gas supply portion 129, or different gases may be supplied.

### [Third Embodiment]

The above second embodiment has shown the example where one gas mist generator 120 is connected with one inhalation member 130, and the present embodiment will show a structure of connecting a plurality of inhalation members.

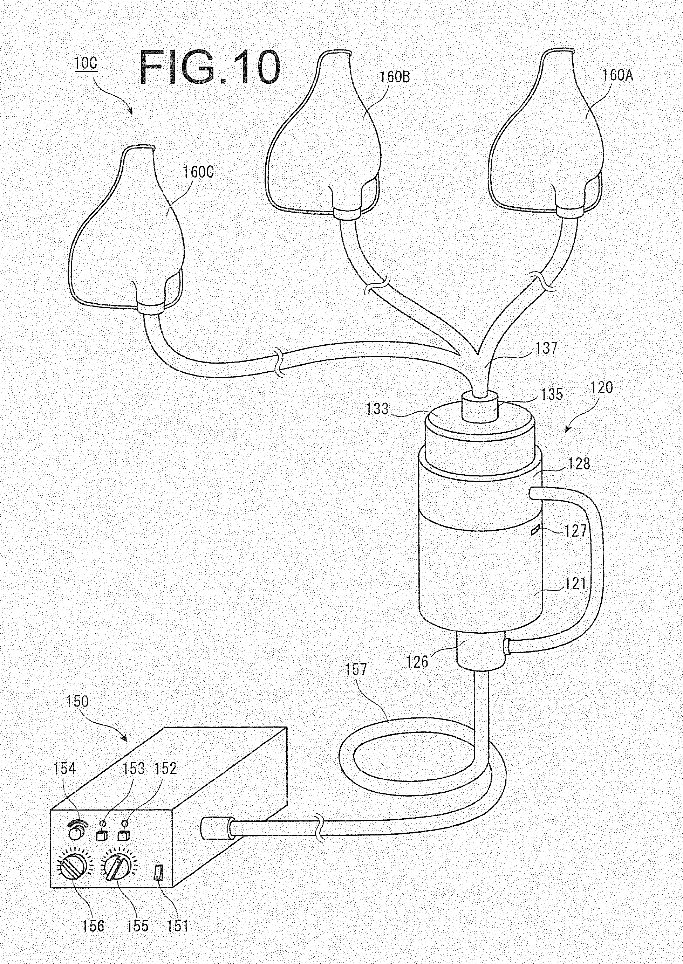

FIG. 10 is the generally schematic view of the gas mist inhaler depending on the third embodiment of this invention. As to the same parts as those of the embodiment shown in FIG.s 1 to 9, the same numerals will be given, and detailed explanation will be omitted.

As shown in FIG. 10, the gas mist inhaler 10C has a gas supply device (gas supply means) 150, the gas mist generator (gas mist generating means) 120 and plural inhalation members (inhalation masks) 160.

The present embodiment equips a gas mist supply pipe 137 diverging into a plurality of pipes (herein, three) between the gas mist generator 120 and a plurality (herein, three) of inhalation members 160A, 160B, 160C. It is thereby possible to connect the plurality (herein, three) of inhalation members 160A, 160B, 160C to one gas supply device 150 and the gas mist generator 120. At this time, the gas supply device 150 adjusts the supplying pressure by the gas supply pressure adjusting part 156 such that a gas inhalation can be performed optimally in each of the plural inhalation members 160A, 160B, 160C.

The present embodiment has illustrated the example using the gas mist supply pipes 137 diverging into the plurality of pipes, and the gas mist outlet 135 may provide plural outlets, each of which is connected with the gas mist pipe, or provide diverged plural pipes on the way of the ordinary gas mist pipes for supplying the gas mist into the plural inhalation members.

As having above mentioned, according to the gas mist inhaler of the present invention, in addition to ordinary effects of the inhaler, and owing to the physiological action of the gas mist, not only permeating the liquid medicine into the upper and lower airways of the living organism, but also activating a blood flow around a diseased part, the invention can display effects such as rapidly moderating an inflammation or increasing immunological force.

The above references have explained the embodiments of the invention, but are not limited thereto, and so far as not deviating from the subject matter of the invention, various kinds of embodiments are, of course, available.

### Industrial Applicability

The present invention is concerned with the gas mist inhalers for carrying out the oral inhalation of the gas mist into the living organism, and has the industrial applicability.

### Reference Signs List

- 10A, 10B, 10C:: gas mist inhalers
- 110:: gas bomb
- 120:: gas mist generator
- 121:: generator main body
- 122:: liquid storage
- 122A:: shielding plate
- 123:: nozzle
- 123A:: nozzle front end opening
- 124:: liquid suction pipe forming member
- 124A:: liquid suction pipe
- 124B:: liquid suction pipe front end portion
- 125:: baffle
- 126:: gas bomb connecting portion
- 126A:: dial switch
- 126B:: residual gage
- 127:: air hole
- 128:: middle member
- 129:: gas supply portion
- 131:: gas inlet
- 132:: gas mist collector
- 133:: cover member
- 134:: gas mist leading part
- 135:: gas mist outlet
- 136:: gas mist supply pipe
- 136A:: cornice shaped pipe
- 137:: gas mist supply pipe
- 140:: inhalation mask
- 140A:: mouth mask
- 140B:: nose mask
- 141, 141A, 141B:: connecting portions
- 142, 142A, 142B:: inhalation ports
- 150:: gas supply device
- 151:: power supply switch
- 152:: oxygen supply ON/OFF switch
- 153:: carbon dioxide supply ON/OFF switch
- 154:: gas mixing ratio setting part
- 155:: OFF timer
- 156:: gas supply pressure adjusting part
- 157:: gas supply pipe
- 160:: plural inhalation members
- 160A, 160B, 160C:: inhalation members

## Claims

1. A gas mist inhaler, comprising
a gas supply means of supplying oxygen, carbon dioxide, or mixed gas (called as "gas" hereafter) of oxygen and carbon dioxide,
a gas mist generation means connected to the gas supply means as well as having a liquid storage of storing a liquid, a nozzle of supplying gas under pressure, a liquid suction pipe of sending the liquid to the front end of the nozzle, and an air hole of taking in outside air, the above mentioned gas mist generation means of generating a mist (called as "gas mist" hereafter) prepared by pulverizing and dissolving the liquid and gas, and
furnishing an inhalation member connected to the gas mist generation means and having an inhalation port of inhaling the gas mist into the living organism,
the above mentioned gas mist generation means further having a gas introduction means of supplying gas into the gas mist generation, independently of the above mentioned nozzle,
wherein supplying pressure of the gas mist into the inhalation member is heightened.

2. The gas mist inhaler as set forth in claim 1, wherein the inhalation member has a further opening for taking in outside air.

3. The gas mist inhaler as set forth in claim 1 or 2, wherein the gas supply means is a gas bomb of a cartridge system.

4. The gas mist inhaler as set forth in any of claims 1 to 3, wherein the gas supply means has any one or plurality of a gas supply time setting portion, a gas supply pressure adjusting portion and a gas mixing ratio setting portion.

5. The gas mist inhaler as set forth in any of claims 1 to 4, wherein the gas mist generation means supplies the gas mist into the plurality of inhalation members.

6. The gas mist inhaler as set forth in claim 1, wherein the above mentioned liquid is any one or plural combination of water, ionic water, ozone water, physiological salt solution, purified water, or sterilized and purified water.

7. The gas mist inhaler as set forth in claim 6, wherein the above mentioned liquid further includes any one or plural combination of menthol, vitamin E, vitamin C derivative, retinol, anesthetic agent, cyclodextrin, photocatalyst, complex of photocatalyst and apatite, hyaluronic acid, coenzyme Q10, seed oil, propolith, ethanol, chlorhexidine gluconate, amphoteric surface active agent, benzalkonium chloride, alkyldiamino etherglycine acetate, sodium hypochlorite, peracetic acid, sodium sesquicarbonate, silica, povidone-iodine, sodium hydrogen carbonate, carbonate spring agent of high density, anti-allergic agent, anti-inflammatory agent, anti-febrile agent, anti-fungus agent, anti-influenza virus agent, influenza vaccine, steroid agent, anti-cancer agent, anti-hypertensive agent, cosmetic, and tricogen.

8. The gas mist inhaler as set forth in claim 1, wherein a size of the mist supplied from the gas mist generating means into the inhalation member is not larger than 10 µm.

9. The gas mist inhaler as set forth in claim 1, wherein the gas mist generating means has a gas mist supply pipe for supplying the gas mist into the inhalation member, and
this gas mist supply pipe is furnished with a filter for removing liquid drops attaching to the inside of the pipe.

10. The gas mist inhaler as set forth in claim 1, wherein the gas mist generating means has the gas mist supply pipe for supplying the gas mist into the inhalation member, and
this gas mist supply pipe is composed of a cornice shaped pipe over a whole or at one part of the gas mist supply pipe.

11. The gas mist inhaler as set forth in claim 1, wherein the gas mist generating means has the gas mist supply pipe for supplying the gas mist into the inhalation member, and
this gas mist supply pipe is furnished with a check valve.

12. The gas mist inhaler as set forth in claim 1, wherein the gas mist generating means is in advance sterilized.
